# EUROPEAN PATENT SPECIFICATION

(11) **EP 3 716 936 B1**
(45) Date of publication and mention of the grant of the patent: **29.10.2025**
(21) Application number: 17829742.0
(22) Date of filing: 01.12.2017
(51) Int. Cl.: A61K 8/27, A61K 8/34, A61K 8/44, A61Q 11/00

(54) **RHEOLOGICAL STABILIZATION SYSTEMS IN A ZINC TOOTHPASTE**
RHEOLOGISCHE STABILISIERUNGSSYSTEME IN EINER ZINKZAHNPASTE
SYSTEMES DE STABILISATION RHEOLOGIQUE DANS UN DENTIFRICE A BASE DE ZINC

(43) Date of publication of application: 07.10.2020
(73) Proprietor: Colgate-Palmolive Company, New York, NY 10022 (US)
(72) Inventor: PATEL, Neeta Atul, Monmouth Junction, New Jersey 08852 (US); SZEWCZYK, Gregory, Flemington, New Jersey 08822 (US); MORGAN, Andre, Robbinsville, New Jersey 08690 (US); MUIR, Melissa, Lopatcong, New Jersey 08865 (US)
(74) Representative: Wibbelmann, Jobst
(86) International application number: PCT/US2017/064147
(87) International publication number: WO 2019/108218

(56) References cited:
- WO-A1-2014/098826
- WO-A1-2015/195124
- US-A1- 2017 172 875
- DATABASE GNPD [online] MINTEL; 2 May 2017 (2017-05-02), ANONYMOUS: "Leports SPF 50+ PA++++", XP055783405, retrieved from https://www.gnpd.com/sinatra/recordpage/4685559/ Database accession no. 4685559

## Description

### FIELD OF THE DISCLOSURE

This disclosure relates to oral care compositions comprising a zinc amino acid halide complex (ZLC), and a humectant system which provides improved rheological stability. Methods of making and using the compositions are also provided.

### BACKGROUND

Polyethylene glycol (PEG) and propylene glycol (PG) are very commonly used humectants/dispersants in toothpaste compositions because of their solubility in water and flexibility as great binders without having significant impact on aesthetic attributes such as foam. Often a mixed humectant system consisting of PEG and PG will be employed to maintain a toothpaste formula free from crystallization. These humectants have previously never been demonstrated to have any interaction or impact the toothpaste compositions rheologically. Nevertheless, the present disclosure has demonstrated their significant impact on rheology of the toothpaste compositions. Experiments indicate that PEG and PG do not remain innocuous in the formulation but rather interact strongly with the zinc species and water present to impact rheology of the toothpaste compositions such as viscosity. The interaction of PEG and PG with zinc and water surprisingly results in improved rheology, texture of the compositions, and thereby, stability of the compositions.

### BRIEF SUMMARY

While oral care compositions comprising the ZLC complex are known, it has been challenging to formulate compositions comprising this complex which have good long-term stability and yield stress. However, the present invention has surprisingly improved stability of and yield stress of toothpaste compositions comprising the ZLC complex by incorporation of a humectant system that include PEG and PG as structuring humectant.

The present disclosure concerns a use of PEG 600 in combination with PG in respective amounts for improving the rheological stability of a toothpaste composition , wherein the composition comprises
a zinc lysine chloride complex, and
PEG 600 in an amount of 2-4% by weight of the toothpaste composition and
PG in an amount of 3-5% by weight of the toothpaste composition,
wherein the composition comprises an organic or inorganic acid to reduce the pH, selected from one or more of phosphoric acid, hydrochloric acid, sulfuric acid, citric acid, tartaric acid, malic acid, maleic acid, fumaric acid, lactic acid, gluconic acid and benzoic acid, in an amount of from 0.05 to 5% by weight of the toothpaste composition.

Further embodiments of the invention are set forth in the attached claims.

Further areas of applicability of the present invention will become apparent from the detailed description provided hereinafter. It should be understood that the detailed description and specific examples, while indicating the preferred embodiment of the invention, are intended for purposes of illustration only and are not intended to limit the scope of the invention.

### DETAILED DESCRIPTION

The following description of the preferred embodiment(s) is merely exemplary in nature and is in no way intended to limit the invention, its application, or uses.

The zinc-amino acid complexes described herein are disclosed in U.S. Patent Application Publication 2015/0328118 and PCT Publication WO 2014/098826.

The zinc cation may complex with two amino acid residues and two chloride residues. For example, where the amino acid is lysine, the complex has the formula [Zn(C₆H₁₄N₂O₂)₂Cl]⁺Cl⁻. In this complex, Zn cation is coordinated by two lysine ligands with two N atoms from NH₂ groups and O atoms from carboxylic groups in an equatorial plane. It displays a distorted square-pyramidal geometry with the apical position occupied by a Cl atom. This novel structure gives rise to a positive cation moiety, to which a Cl⁻ anion is combined to form an ionic salt.

Other complexes of zinc and amino acid are possible, and the precise form is dependent in part on the molar ratios of the precursor compounds, e.g., if there is limited halide, halide-free complexes may form, e.g. ZnOLys₂, having a pyramid geometry, with the equatorial plane that is same as the above compound (Zn is bound to two oxygen and two nitrogen atoms from different lysines), wherein the top of the pyramid is occupied by an O atom.

Mixtures of complexes and/or additional complex structures, e.g., involving multiple zinc ions based on the zinc structure, are possible and contemplated within the scope of the invention. When the complexes are in solid form, they may form crystals, e.g. in hydrated form.

Irrespective of the precise structure of the complex or complexes, however, the interaction of the zinc and the amino acid converts insoluble zinc oxide or zinc salts to a highly soluble complex at approximately neutral pH. With increasing dilution in water, however, the complex disassociates, and the zinc ion converts to insoluble zinc oxide. This precipitation occludes the dentinal tubules, thereby reducing hypersensitivity, and also provides zinc to the enamel, which reduces acid erosion, biofilm and plaque formation.

. The active zinc-amino acid complex is provided in a toothpaste. Upon brushing, the active is diluted by saliva and water, leading to precipitation and the formation of deposits and occluding particles.

The zinc ion source for combination with an amino acid halide or an amino acid optionally plus halogen acid in this case may be, e.g., zinc oxide or zinc chloride.

In certain embodiments, the amount of zinc amino acid halide complex in the composition is 0.05 to 10% by weight of the composition. In certain embodiments, precursors, e.g., zinc and amino acid hydrohalide, are present in amounts such that when combined into the zinc amino acid halide, the zinc amino acid halide would be present in an amount of 0.05 to 10 % by weight of the composition. In either of these embodiments, the amount of the zinc amino acid halide can be varied for the desired purpose, such as a dentifrice or a mouthwash. In other embodiments, the amount of the zinc amino acid halide is at least 0.1, at least 0.2, at least 0.3, at least 0.4, at least 0.5, at least 1, at least 2, at least 3, or at least 4 up to 10% by weight of the composition. In other embodiments, the amount of the zinc amino acid halide is less than 9, less than 8, less than 7, less than 6, less than 5, less than 4, less than 3, less than 2, less than 1, less than 0.5 to 0.05 % by weight of the composition. In other embodiments, the amounts are 0.05 to 5%, 0.05 to 4%, 0.05 to 3%, 0.05 to 2%, 0.1 to 5%, 0.1 to 4%, 0.1 to 3%, 0.1 to 2%, 0.5 to 5%, 0.5 to 4%, 0.5 to 3%, or 0.5 to 2% by weight of the composition.

In certain embodiments, zinc is present in an amount of 0.05 to 10% by weight of the composition. In other embodiments, the amount of zinc is at least 0.1, at least 0.2, at least 0.3, at least 0.4, at least 0.5, at least 1, at least 2, at least 3, or at least 4 up to 10% by weight of the composition. In other embodiments, the amount of the zinc is less than 9, less than 8, less than 7, less than 6, less than 5, less than 4, less than 3, less than 2, less than 1, less than 0.5 to 0.05 % by weight of the composition. In other embodiments, the amounts are 0.05 to 5%, 0.05 to 4%, 0.05 to 3%, 0.05 to 2%, 0.1 to 5%, 0.1 to 4%, 0.1 to 3%, 0.1 to 2%, 0.5 to 5%, 0.5 to 4%, 0.5 to 3%, or 0.5 to 2% by weight of the composition.

In certain embodiments, amino acid hydrohalide is present in an amount of 0.05 to 30% by weight. In other embodiments, the amount is at least 0.1, at least 0.2, at least 0.3, at least 0.4, at least 0.5, at least 1, at least 2, at least 3, at least 4, at least 5, at least 10, at least 15, at least 20 up to 30% by weight. In other embodiments, the amount is less than 30, less than 25, less than 20, less than 15, less than 10, less than 5, less than 4, less than 3, less than 2, or less than 1 down to 0.05% by weight of the composition.

Where precursor materials are present, they are preferably present in molar ratios approximately as required to produce the desired zinc amino acid halide, although an excess of one material or another may be desirable in certain formulations, e.g., to balance pH against other formulation constituents, to provide additional antibacterial zinc, or to provide amino acid buffer. Preferably, however, the amount of halide is limited, as constraining the level of halide somewhat encourages interaction between the zinc and the amino acid.

In some embodiments, the total amount of zinc in the composition is 0.05 to 8 % by weight of the composition. In other embodiments, the total amount of zinc is at least 0.1, at least 0.2, at least 0.3, at least 0.4, at least 0.5, or at least 1 up to 8% by weight of the composition. In other embodiments, the total amount of zinc in the composition is less than 5, less than 4, less than 3, less than 2, or less than 1 to 0.05% by weight of the composition, e.g. about 0.84%.

In certain embodiments, a molar ratio of zinc to amino acid is at least 2:1. In other embodiments, the molar ratio is at least 1:1, at least 1:2, at least 1:3, at least 1:4, 2:1 to 1:4, 1:1 to 1:4, 1:2 to 1:4, 1:3 to 1:4, 2:1 to 1:3, 2:1 to 1:2, 2:1 to 1:1, or 1:3. Above 1:4, it is expected that the zinc will be totally dissolved.

The carrier represents all other materials in the composition other than the zinc amino acid halide complex or its precursors. The amount of carrier is then the amount to reach 100% by adding to the weight of the zinc amino acid halide, including any precursors.

*Active Agents*: The compositions disclosed herein may comprise various agents which are active to protect and enhance the strength and integrity of the enamel and tooth structure and/or to reduce bacteria and associated tooth decay and/or gum disease, including or in addition to the zinc - amino acid - halide complexes. Effective concentration of the active ingredients used herein will depend on the particular agent and the delivery system used. It is understood that a toothpaste for example will typically be diluted with water upon use, while a mouth rinse typically will not be. The concentration will also depend on the exact salt or polymer selected. For example, where the active agent is provided in salt form, the counterion will affect the weight of the salt, so that if the counterion is heavier, more salt by weight will be required to provide the same concentration of active ion in the final product. Arginine, where present, may be present at levels from, e.g., about 0.1 to about 20 wt % (expressed as weight of free base), e.g., about 1 to about 10 wt % for a consumer toothpaste or about 7 to about 20 wt % for a professional or prescription treatment product. Fluoride where present may be present at levels of, e.g., about 25 to about 25,000 ppm, for example about 750 to about 2,000 ppm for a consumer toothpaste, or about 2,000 to about 25,000 ppm for a professional or prescription treatment product. Levels of antibacterial agents will vary similarly, with levels used in toothpaste being e.g., about 5 to about 15 times greater than used in mouthrinse. For example, a triclosan toothpaste may contain about 0.3 wt % triclosan.

*Fluoride Ion Source*: The oral care compositions may further include one or more fluoride ion sources, e.g., soluble fluoride salts. A wide variety of fluoride ion-yielding materials can be employed as sources of soluble fluoride in the present compositions. Representative fluoride ion sources include, but are not limited to, stannous fluoride, sodium fluoride, potassium fluoride, sodium monofluorophosphate, sodium fluorosilicate, ammonium fluorosilicate, amine fluoride, ammonium fluoride, and combinations thereof. In certain embodiments, the fluoride ion source includes stannous fluoride, sodium fluoride, sodium monofluorophosphate as well as mixtures thereof. In certain embodiments, the oral care composition of the invention may also contain a source of fluoride ions or fluorine-providing ingredient in amounts sufficient to supply about 25 ppm to about 25,000 ppm of fluoride ions, generally at least about 500 ppm, e.g., about 500 to about 2000 ppm, e.g., about 1000 to about 1600 ppm, e.g., about 1450 ppm. The appropriate level of fluoride will depend on the particular application. A toothpaste for general consumer use would typically have about 1000 to about 1500 ppm, with pediatric toothpaste having somewhat less. A dentifrice or coating for professional application could have as much as about 5,000 or even about 25,000 ppm fluoride. Fluoride ion sources may be added to the compositions of the invention at a level of about 0.01 wt. % to about 10 wt. % in one embodiment, or about 0.03 wt. % to about 5 wt. % in another embodiment, or about 0.1 wt. % to about 1 wt. % by weight of the composition in another embodiment. Weights of fluoride salts to provide the appropriate level of fluoride ion will obviously vary based on the weight of the counterion in the salt. In one embodiment, the composition contains sodium fluoride as a fluoride source in an amount of 0.03 % to 5%, or 0.1 % to 1 % by weight of the composition, or about 0.32% by weight of the composition.

*Abrasives*: The compositions disclosed herein, may include silica abrasives, and may comprise additional abrasives, e.g., a calcium phosphate abrasive, e.g., tricalcium phosphate (Ca₃(PO₄)₂), hydroxyapatite (Ca₁₀(PO₄)₆(OH)₂), or dicalcium phosphate dihydrate (CaHPO₄ • 2H₂O, also sometimes referred to herein as DiCal) or calcium pyrophosphate; calcium carbonate abrasive; or abrasives such as sodium metaphosphate, potassium metaphosphate, aluminum silicate, calcined alumina, bentonite or other siliceous materials, or combinations thereof.

Other silica abrasive polishing materials useful herein, as well as the other abrasives, generally have an average particle size ranging between about 0.1 and about 30 microns, about between 5 and about 15 microns. The silica abrasives can be from precipitated silica or silica gels, such as the silica xerogels. Particular silica xerogels are marketed under the trade name Syloid^{®} by the W. R. Grace & Co., Davison Chemical Division. The precipitated silica materials include those marketed by the J. M. Huber Corp. under the trade name Zeodent^{®}, including the silica carrying the designation Zeodent 114 and 165. In certain embodiments, abrasive materials useful in the practice of the oral care compositions in accordance with the invention include silica gels and precipitated amorphous silica having an oil absorption value of less than about 100 cc/100 g silica and in the range of about 45 cc/100 g to about 70 cc/100 g silica. Oil absorption values are measured using the ASTA Rub-Out Method D281. In certain embodiments, the silicas are colloidal particles having an average particle size of about 3 microns to about 12 microns, and about 5 to about 10 microns. Low oil absorption silica abrasives particularly useful in the practice of the invention are marketed under the trade designation Sylodent XWA^{®} by Davison Chemical Division of W.R. Grace & Co., Baltimore, Md. 21203. Sylodent 650 XWA^{®}, a silica hydrogel composed of particles of colloidal silica having a water content of 29% by weight averaging about 7 to about 10 microns in diameter, and an oil absorption of less than about 70 cc/100 g of silica is an example of a low oil absorption silica abrasive useful in the practice of the present invention. In one embodiment, the compositions contain

*Foaming agents:* The oral care compositions disclosed herein also may include an agent to increase the amount of foam that is produced when the oral cavity is brushed. Illustrative examples of agents that increase the amount of foam include, but are not limited to polyoxyethylene and certain polymers including, but not limited to, alginate polymers. The polyoxyethylene may increase the amount of foam and the thickness of the foam generated by the oral care carrier component of the present invention. Polyoxyethylene is also commonly known as polyethylene glycol ("PEG") or polyethylene oxide. The polyoxyethylenes suitable for this invention will have a molecular weight of about 200,000 to about 7,000,000. In one embodiment the molecular weight will be about 600,000 to about 2,000,000 and in another embodiment about 800,000 to about 1,000,000. Polyox^{®} is the trade name for the high molecular weight polyoxyethylene produced by Union Carbide. The polyoxyethylene may be present in an amount of about 1% to about 90%, in one embodiment about 5% to about 50% and in another embodiment about 10% to about 20% by weight of the oral care carrier component of the oral care compositions of the present invention. Where present, the amount of foaming agent in the oral care composition (i.e., a single dose) is about 0.01 to about 0.9 % by weight, about 0.05 to about 0.5% by weight, and in another embodiment about 0.1 to about 0.2 % by weight.

*Surfactants*: The compositions disclosed herein may contain anionic surfactants, for example:
i. water-soluble salts of higher fatty acid monoglyceride monosulfates, such as the sodium salt of the monosulfated monoglyceride of hydrogenated coconut oil fatty acids such as sodium N-methyl N-cocoyl taurate, sodium cocomonoglyceride sulfate,
ii. higher alkyl sulfates, such as sodium lauryl sulfate,
iii. higher alkyl-ether sulfates, e.g., of formula CH₃(CH₂)ₘCH₂(OCH₂CH₂)ₙOSO₃X, wherein m is 6-16, e.g., 10, n is 1-6, e.g., 2, 3 or 4, and X is Na or K, for example sodium laureth-2 sulfate (CH₃(CH₂)₁₀CH₂(OCH₂CH₂)₂OSO₃Na).
iv. higher alkyl aryl sulfonates such as sodium dodecyl benzene sulfonate (sodium lauryl benzene sulfonate)
v. higher alkyl sulfoacetates, such as sodium lauryl sulfoacetate (dodecyl sodium sulfoacetate), higher fatty acid esters of 1,2 dihydroxy propane sulfonate, sulfocolaurate (N-2-ethyl laurate potassium sulfoacetamide) and sodium lauryl sarcosinate.

By "higher alkyl" is meant, e.g., C₆₋₃₀ alkyl. In particular embodiments, the anionic surfactant is selected from sodium lauryl sulfate and sodium ether lauryl sulfate. The anionic surfactant may be present in an amount which is effective, e.g., > 0.01% by weight of the formulation, but not at a concentration which would be irritating to the oral tissue, e.g., <10%, and optimal concentrations depend on the particular formulation and the particular surfactant. For example, concentrations used or a mouthwash are typically on the order of one tenth that used for a toothpaste. In one embodiment, the anionic surfactant is present in a toothpaste at from about 0.3% to about 4.5% by weight, e.g., about 1.5%. The compositions of the invention may optionally contain mixtures of surfactants, e.g., comprising anionic surfactants and other surfactants that may be anionic, cationic, zwitterionic or nonionic. Generally, surfactants are those which are reasonably stable throughout a wide pH range. In certain embodiments, the anionic surfactants useful herein include the water-soluble salts of alkyl sulfates having about 10 to about 18 carbon atoms in the alkyl radical and the water-soluble salts of sulfonated monoglycerides of fatty acids having about 10 to about 18 carbon atoms. Sodium lauryl sulfate, sodium lauroyl sarcosinate and sodium coconut monoglyceride sulfonates are examples of anionic surfactants of this type. In a particular embodiment, the composition of the invention, e.g., Composition 1, et seq., comprises sodium lauryl sulfate.

The surfactant or mixtures of compatible surfactants can be present in the compositions of the present invention in about 0.1% to about 5.0%, in another embodiment about 0.3% to about 3.0% and in another embodiment about 0.5% to about 2.0% by weight of the total composition.

*Tartar control agents:* In various embodiments, the compositions disclosed herein may comprise an anticalculus (tartar control) agent. Suitable anticalculus agents include without limitation phosphates and polyphosphates (for example pyrophosphates), polyaminopropanesulfonic acid (AMPS), hexametaphosphate salts, zinc citrate trihydrate, polypeptides, polyolefin sulfonates, polyolefin phosphates, diphosphonates. The invention thus may comprise phosphate salts. In particular embodiments, these salts are alkali phosphate salts, i.e., salts of alkali metal hydroxides or alkaline earth hydroxides, for example, sodium, potassium or calcium salts. "Phosphate" as used herein encompasses orally acceptable mono- and polyphosphates, for example, P₁₋₆ phosphates, for example monomeric phosphates such as monobasic, dibasic or tribasic phosphate; dimeric phosphates such as pyrophosphates; and multimeric phosphates, e.g., sodium hexametaphosphate. In particular examples, the selected phosphate is selected from alkali dibasic phosphate and alkali pyrophosphate salts, e.g., selected from sodium phosphate dibasic, potassium phosphate dibasic, dicalcium phosphate dihydrate, calcium pyrophosphate, tetrasodium pyrophosphate, tetrapotassium pyrophosphate, sodium tripolyphosphate, and mixtures of any of two or more of these. In a particular embodiment, for example the compositions comprise a mixture of tetrasodium pyrophosphate (Na₄P₂O₇), calcium pyrophosphate (Ca₂P₂O₇), and sodium phosphate dibasic (Na₂HPO₄), e.g., in amounts of ca. 3-4% of the sodium phosphate dibasic and ca. 0.2-1% of each of the pyrophosphates. In another embodiment, the compositions comprise a mixture of tetrasodium pyrophosphate (TSPP) and sodium tripolyphosphate (STPP)(Na₅P₃O₁₀), e.g., in proportions of TSPP at about 1-2% and STPP at about 7% to about 10%. Such phosphates are provided in an amount effective to reduce erosion of the enamel, to aid in cleaning the teeth, and/or to reduce tartar buildup on the teeth, for example in an amount of 2-20%, e.g., ca. 5-15%, by weight of the composition.

*Flavoring Agents*: The oral care compositions disclosed herein may also include a flavoring agent. Flavoring agents which are used in the practice of the present invention include, but are not limited to, essential oils as well as various flavoring aldehydes, esters, alcohols, and similar materials. Examples of the essential oils include oils of spearmint, peppermint, wintergreen, sassafras, clove, sage, eucalyptus, marjoram, cinnamon, lemon, lime, grapefruit, and orange. Also useful are such chemicals as menthol, carvone, and anethole. Certain embodiments employ the oils of peppermint and spearmint. The flavoring agent may be incorporated in the oral composition at a concentration of about 0.1 to about 5% by weight e.g. about 0.5 to about 1.5% by weight.

*Polymers*: The oral care compositions disclosed herein may also include additional polymers to adjust the viscosity of the formulation or enhance the solubility of other ingredients. Such additional polymers include polysaccharides (e.g., cellulose derivatives, for example carboxymethyl cellulose, or polysaccharide gums, for example xanthan gum or carrageenan gum), and polyvinyl pyrrolidone. Acidic polymers, for example polyacrylate gels, may be provided in the form of their free acids or partially or fully neutralized water soluble alkali metal (e.g., potassium and sodium) or ammonium salts.

Silica thickeners, which form polymeric structures or gels in aqueous media, may be present. Note that these silica thickeners are physically and functionally distinct from the particulate silica abrasives also present in the compositions, as the silica thickeners are very finely divided and provide little or no abrasive action. Other thickening agents are carboxyvinyl polymers, carrageenan, hydroxyethyl cellulose and water-soluble salts of cellulose ethers such as sodium carboxymethyl cellulose and sodium carboxymethyl hydroxyethyl cellulose. Natural gums such as karaya, gum arabic, and gum tragacanth can also be incorporated. Colloidal magnesium aluminum silicate can also be used as component of the thickening composition to further improve the composition's texture. In certain embodiments, thickening agents in an amount of 0.5% to 5.0% by weight of the total composition are used.

The compositions disclosed herein may include an anionic polymer, for example in an amount of from about 0.05 to about 5%. Such agents are known generally for use in dentifrice, although not for this particular application, useful in the present invention are disclosed in U.S. Pat. Nos. 5,188,821 and 5,192,531; and include synthetic anionic polymeric polycarboxylates, such as 1:4 to 4:1 copolymers of maleic anhydride or acid with another polymerizable ethylenically unsaturated monomer, preferably methyl vinyl ether/maleic anhydride having a molecular weight (M.W.) of about 30,000 to about 1,000,000, most preferably about 300,000 to about 800,000. These copolymers are available for example as Gantrez. e.g., AN 139 (M.W. 500,000), AN 119 (M.W. 250,000) and preferably S-97 Pharmaceutical Grade (M.W. 700,000) available from ISP Technologies, Inc., Bound Brook, N.J. 08805. The enhancing agents when present are present in amounts ranging from about 0.05 to about 3% by weight. Other operative polymers include those such as the 1:1 copolymers of maleic anhydride with ethyl acrylate, hydroxyethyl methacrylate, N-vinyl-2-pyrollidone, or ethylene, the latter being available for example as Monsanto EMA No. 1103, M.W. 10,000 and EMA Grade 61, and 1:1 copolymers of acrylic acid with methyl or hydroxyethyl methacrylate, methyl or ethyl acrylate, isobutyl vinyl ether or N-vinyl-2-pyrrolidone. Suitable generally, are polymerized olefinically or ethylenically unsaturated carboxylic acids containing an activated carbon-to-carbon olefinic double bond and at least one carboxyl group, that is, an acid containing an olefinic double bond which readily functions in polymerization because of its presence in the monomer molecule either in the alpha-beta position with respect to a carboxyl group or as part of a terminal methylene grouping. Illustrative of such acids are acrylic, methacrylic, ethacrylic, alpha-chloroacrylic, crotonic, beta-acryloxy propionic, sorbic, alpha-chlorsorbic, cinnamic, beta-styrylacrylic, muconic, itaconic, citraconic, mesaconic, glutaconic, aconitic, alpha-phenylacrylic, 2-benzyl acrylic, 2-cyclohexylacrylic, angelic, umbellic, fumaric, maleic acids and anhydrides. Other different olefinic monomers copolymerizable with such carboxylic monomers include vinylacetate, vinyl chloride, dimethyl maleate and the like. Copolymers contain sufficient carboxylic salt groups for water-solubility. A further class of polymeric agents includes a composition containing homopolymers of substituted acrylamides and/or homopolymers of unsaturated sulfonic acids and salts thereof, in particular where polymers are based on unsaturated sulfonic acids selected from acrylamidoalykane sulfonic acids such as 2-acrylamide 2 methylpropane sulfonic acid having a molecular weight of about 1,000 to about 2,000,000. Another useful class of polymeric agents includes polyamino acids containing proportions of anionic surface-active amino acids such as aspartic acid, glutamic acid and phosphoserine.

The term average molecular weight of a polymer is the total weight of its sample divided by the number of molecules in the sample, i.e., ΣNᵢMᵢ/ ΣNᵢ, and those skilled in the art can readily calculate the value using molecular weight values determined by size exclusion chromatograph or MALDI mass spectrometry.

*Water*: The oral compositions may comprise significant levels of water. Water employed in the preparation of commercial oral compositions should be deionized and free of organic impurities. The amount of water in the compositions includes the free water which is added plus that amount which is introduced with other materials.

*Humectants:* Within certain embodiments of the oral compositions, it is also desirable to incorporate a humectant to prevent the composition from hardening upon exposure to air. Certain humectants can also impart desirable sweetness or flavor to the compositions. Suitable humectants include edible polyhydric alcohols such as polyethylene glycol, e.g., PEG 600, glycerine, sorbitol, xylitol, propylene glycol as well as other polyols and mixtures of these humectants. In one embodiment of the disclosure, the oral compositions contain a structuring humectant, wherein the structuring humectant is PEG and PG, which are both present in an amount of 1-10% by weight of the composition. Preferably, PEG is present in an amount of 2-4% by weight of the composition and PG in an amount of 3-5% by weight of the composition. More preferably, PEG is present in an amount of about 3% by weight of the composition and PG in an amount of about 4% by weight of the composition.

*Other optional ingredients:* In addition to the above-described components, the embodiments of the compositions disclosed herein can contain a variety of optional dentifrice ingredients some of which are described below. Optional ingredients include, for example, but are not limited to, adhesives, sudsing agents, flavoring agents, sweetening agents, additional antiplaque agents, abrasives, and coloring agents.

Unless stated otherwise, all percentages of composition components given in this specification are by weight based on a total composition or formulation weight of 100%.

The compositions and formulations as provided herein are described and claimed with reference to their ingredients, as is usual in the art. As would be evident to one skilled in the art, the ingredients may in some instances react with one another, so that the true composition of the final formulation may not correspond exactly to the ingredients listed. Thus, it should be understood that the invention extends to the product of the combination of the listed ingredients.

As used throughout, ranges are used as shorthand for describing each and every value that is within the range. Any value within the range can be selected as the terminus of the range. In addition, all references cited herein are hereby incorporated by referenced in their entireties. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material.

### EXAMPLES

U.S. Patent Application Publication 2015/0328118 and PCT Publication WO 2014/098826 present experimental data demonstrating the ability of oral care compositions comprising ZLC to precipitate insoluble zinc compounds and to occlude dentinal tubules, thus making them useful in the treatment of dentinal hypersensitivity.

Several test toothpaste compositions comprising zinc-lysine complex (ZLC) are prepared and evaluated in room temperature aging studies and accelerated aging studies. After the designated period of time at the selected temperature, the viscosity of the compositions is measured by determining the yield stress of the compositions. The viscosity is measured by a Brookfield Viscometer using SpindleV74. Tables 1 - 4 show the compositions tested, and Tables 5-8 show test results.

Control toothpaste Formula 1 containing neither PEG 600 nor PG is shown in Table 1.

**Table 1**

| **Ingredients** | Formula 1 (wt %) |
|---|---|
| Demineralized Water | Balance |
| Sucralose | 0.02 |
| Sodium Saccharin | 0.20 |
| Sodium Fluoride | 0.32 |
| Sodium Tripolyphosphate | 2.00 |
| Glycerin | 47.56 |
| Polyethylene Glycol 600 | 0.00 |
| Propylene Glycol | 0.00 |
| Xanthan Gum | 0.1 |
| Polyvinyl Pyrrolidone | 1.0 |
| Silica Abrasive/cleaning | 22.0 |
| TiO₂ | 0.40 |
| ZLC complex | 5.77 |
| 35% Hydrochloric Acid | 0.68 |
| Sodium Hydroxide (50%) | 0.50 |
| Anionic Surfactant | 1.75 |
| Zwitterionic Surfactant | 1.00 |
| Flavor | 1.70 |
| Total Components | 100.00 |

Toothpaste Formulae 4-6 containing no PG are shown in Table 3.

**Table 3**

| **Ingredients** | **Formula 4 (wt%)** | **Formula 5 (wt%)** | **Formula 6 (wt%)** |
|---|---|---|---|
| Demineralized Water | Balance | Balance | Balance |
| Sucralose | 0.02 | 0.02 | 0.02 |
| Sodium Saccharin | 0.20 | 0.20 | 0.20 |
| Sodium Fluoride | 0.32 | 0.32 | 0.32 |
| Sodium Tripolyphosphate | 2.00 | 2.00 | 2.00 |
| Glycerin | 48.31 | 46.81 | 44.31 |
| Polyethylene Glycol 600 | 3.00 | 4.50 | 7.00 |
| Propylene Glycol | 0.00 | 0.00 | 0.00 |
| Xanthan Gum | 0.1 | 0.1 | 0.1 |
| Carboxymethylcelullose Sodium | 0.3 | 0.3 | 0.3 |
| Polyvinyl Pyrrolidone | 1.25 | 1.25 | 1.25 |
| Silica Abrasive/cleaning | 18.0 | 18.0 | 18.0 |
| TiO₂ | 0.40 | 0.40 | 0.40 |
| 85% Syrup phosphoric acid | 0.20 | 0.20 | 0.20 |
| ZLC complex | 5.77 | 5.77 | 5.77 |
| 35% Hydrochloric Acid | 0.68 | 0.68 | 0.68 |
| Anionic Surfactant | 1.75 | 1.75 | 1.75 |
| Zwitterionic Surfactant | 1.00 | 1.00 | 1.00 |
| Flavor | 1.70 | 1.70 | 1.70 |
| Total Components | 100.00 | 100.00 | 100.00 |

Toothpaste Formula 7 -according to the invention- containing 3% PEG 600 and 4% PG is shown in Table 4.

**Table 4**

| **Ingredients** | **Formula 7 (wt%)** |
|---|---|
| Demineralized Water | Balance |
| Sucralose | 0.02 |
| Sodium Saccharin | 0.20 |
| Sodium Fluoride | 0.32 |
| Sodium Tripolyphosphate | 2.00 |
| Glycerin | 40.56 |
| Polyethylene Glycol 600 | 3.00 |
| Propylene Glycol | 4.00 |
| Xanthan Gum | 0.1 |
| Polyvinyl Pyrrolidone | 1.0 |
| Silica Abrasive/cleaning | 22.0 |
| TiO₂ | 0.40 |
| ZLC complex | 5.77 |
| 35% Hydrochloric Acid | 0.68 |
| Sodium Hydroxide (50%) | 0.50 |
| Anionic Surfactant | 1.75 |
| Zwitterionic Surfactant | 1.00 |
| Flavor | 1.70 |
| Total Components | 100.00 |

Yield stress test results of Formula 1 are shown in Table 5.

**Table 5**

| Duration | Formula 1 | |
|---|---|---|
| | Temperature | Yield Stress cPs at 1 RPM |
| Initial | | 235755 |
| 1 month | CRT | 70851 |
| | 40°C | 47664 |
| | 49°C | 59258 |

Formula 1, which contains neither PEG 600 nor PG, is shown to become less viscous at room temperature, 40°C, and 49°C aging for 1 month, and complete loss of yield stress and stand up within 1 week.

Yield stress test results of Formulae 2 and 3 are shown in Table 6

**Table 6**

| Duration | Temperature | Yield Stress cPs at 1 RPM | |
|---|---|---|---|
| | | Formula 2 | Formula 3 |
| Initial | | 600953 | 533966 |
| 1 month | CRT | 36070 | 302731 |
| | 40°C | 36070 | 111431 |
| | 49°C | 47664 | 105634 |

Like control Formula 1, Formulae 2 and 3, which contain no PEG but contain 4% PG and 7% PG respectively, are shown to become less viscous at room temperature, 40°C, and 49°C aging for 1 month, and complete loss of yield stress and stand up within 1 week.

Yield stress test results of Formulae 4-6 are shown in Table 7.

**Table 7**

| Duration | Temperature | Yield Stress cPs at 1 RPM | |
|---|---|---|---|
| | | Formula 5 | Formula 6 |
| Initial | | 512598 | 61740 |
| 1 week | CRT | 366753 | 111584 |
| 1 month | CRT | | 129428 |
| | 40°C | | 81844 |
| | 49°C | | 81844 |
| 3 months | CRT | | 363239 |
| | 40°C | | 56578 |
| | 49°C | | 50781 |

Formula 4 that contains no PG but 3% PEG shows immediate separation turning in to thin consistency, while Formula 5 that contains no PG but4.5% PEG shows phase separation at room temperature aging for 1 week. However, Formula 6, which contains no PG but 7% PEG, built some viscosity at room temperature aging for 1 week, 1 month and 3 months, and appearance of gel ball texture at 40°C 3 months, demonstrating that higher PEG 600 increases incidence of texture/ gelation look.

The above yield stress test results of Formulae 2-6 show that the removal of either PEG or PG cause complete loss of yield stress (stand up) and viscosity within 1 week at room temperature.

Yield stress test results of Formula 7 are shown in Table 8.

**Table 8**

| Duration | Temperature | Yield Stress cPs at 1 RPM |
|---|---|---|
| Initial | | 269095 |
| 1 month | CRT | 531390 |
| | 40°C | 589359 |
| | 49°C | 552001 |
| 2 months | CRT | 485177 |
| | 40°C | 583562 |
| | 49°C | 497896 |

In contrast with the other formulae, Formula 7, which contains 3% PEG and contains 4% PG, is surprisingly shown to become more viscous across all the tested temperatures-at room temperature, 40°C, 49°C aging for 1 month and 2 months, improved viscosity and a complete retention of the viscosity and yield stress (stand- up).

While the invention has been described with respect to specific examples including presently preferred modes of carrying out the invention, those skilled in the art will appreciate that there are numerous variations and permutations of the above described systems and techniques. It is to be understood that other embodiments may be utilized and structural and functional modifications may be made without departing from the scope of the present invention. Thus, the scope of the invention should be construed broadly as set forth in the appended claims.

## Claims

1. Use of Polyethylene Glycol 600 (PEG 600) in combination with Propylene Glycol in respective amounts for improving the rheological stability of a toothpaste composition , wherein the composition comprises
a zinc lysine chloride complex, and
PEG 600 in an amount of 2-4% by weight of the toothpaste composition and
PG in an amount of 3-5% by weight of the toothpaste composition,
wherein the composition comprises an organic or inorganic acid to reduce the pH, selected from one or more of phosphoric acid, hydrochloric acid, sulfuric acid, citric acid, tartaric acid, malic acid, maleic acid, fumaric acid, lactic acid, gluconic acid and benzoic acid, in an amount of from 0.05 to 5% by weight of the toothpaste composition.

2. The use of claim 1, wherein the composition comprises the acid in an amount of from 0.1 to 2% by weight of the toothpaste composition.

3. The use according to any of the foregoing claims, wherein the lysine in free or in salt form.

4. The use according to any of the foregoing claims, wherein the zinc lysine chloride complex having the chemical structure [Zn(C₆H₁₄N₂O₂)₂Cl]⁺ Cl⁻ ("ZLC") is either in solution of the cationic complex ([Zn(C₆H₁₄N₂O₂)₂Cl]⁺) and the chloride anion, or in solid salt form, optionally in mono- or dihydrate form.

5. The use according to any of the foregoing claims, wherein the zinc amino acid complex is present in an amount of 2-10% by weight of the toothpaste composition.

6. The use according to any of the foregoing claims, wherein the PEG 600 is present in an amount of 3% by weight of the toothpaste composition and the PG in an amount of 4% by weight of the toothpaste composition.

7. The use according to any of the foregoing claims, wherein the composition comprises sodium tripolyphosphate.

8. The use according to any of the foregoing claims, wherein the composition comprises ingredients selected from one or more of abrasives, buffering agents, surfactants, thickeners, gum strips or fragments, breath fresheners, flavoring, fragrance, coloring, antibacterial agents, whitening agents, agents that interfere with or prevents bacterial attachment, calcium sources, orally acceptable potassium salts, and anionic polymers.

9. The use according to any of the foregoing claims, wherein the composition comprises an effective amount of a fluoride ion source.

## Patentansprüche

1. Verwendung von Polyethylenglykol 600 (PEG 600) in Kombination mit Propylenglykol in entsprechenden Mengen zur Verbesserung der rheologischen Stabilität einer Zahnpasta-Zusammensetzung , wobei die Zusammensetzung umfasst
einen Zink-Lysin-Chlorid-Komplex und
PEG 600 in einer Menge von 2 bis 4 Gew.-% der Zahnpastazusammensetzung und
PG in einer Menge von 3 bis 5 Gew.-% der Zahnpastazusammensetzung ,
wobei die Zusammensetzung eine organische oder anorganische Säure zur Senkung des pH-Werts enthält, ausgewählt aus einer oder mehreren der folgenden Säuren: Phosphorsäure, Salzsäure, Schwefelsäure, Zitronensäure, Weinsäure, Apfelsäure, Maleinsäure, Fumarsäure, Milchsäure, Gluconsäure und Benzoesäure, in einer Menge von 0,05 bis 5 Gew.-% der Zahnpastazusammensetzung.

2. Verwendung nach Anspruch 1, wobei die Zusammensetzung die Säure in einer Menge von 0,1 bis 2 Gew.-% der Zahnpastazusammensetzung umfasst.

3. Verwendung gemäß einem der vorstehenden Ansprüche, wobei das Lysin in freier oder in Salzform ist.

4. Verwendung gemäß einem der vorstehenden Ansprüche, wobei der Zink-Lysin-Chlorid-Komplex mit der chemischen Struktur [Zn(C₆H₁₄N₂O₂)₂ Cl]⁺ Cl⁻ ("ZLC") entweder in Lösung des kationischen Komplexes ([Zn(C₆H₁₄N₂O₂)₂ Cl]⁺ ) und dem Chloridanion oder in fester Salzform, gegebenenfalls in Mono- oder Dihydratform, vorliegt.

5. Verwendung gemäß einem der vorstehenden Ansprüche, wobei der Zinkaminosäurekomplex in einer Menge von 2 bis 10 Gew.-% der Zahnpastazusammensetzung vorliegt.

6. Verwendung gemäß einem der vorstehenden Ansprüche, wobei das PEG 600 in einer Menge von 3 Gew.-% der Zahnpastazusammensetzung und das PG in einer Menge von 4 Gew.-% der Zahnpastazusammensetzung vorhanden ist.

7. Verwendung gemäß einem der vorstehenden Ansprüche, wobei die Zusammensetzung Natriumtripolyphosphat umfasst.

8. Verwendung gemäß einem der vorstehenden Ansprüche, wobei die Zusammensetzung Inhaltsstoffe umfasst, die aus einem oder mehreren der folgenden ausgewählt sind: Schleifmittel, Pufferungsmittel, Tenside, Verdickungsmittel, Gummistreifen oder -fragmente, Atemerfrischer, Aromastoffe, Duftstoffe, Farbstoffe, antibakterielle Mittel, Weißmacher, Mittel, die die Anhaftung von Bakterien stören oder verhindern, Calciumquellen, oral verträgliche Kaliumsalze und anionische Polymere.

9. Verwendung gemäß einem der vorstehenden Ansprüche, wobei die Zusammensetzung eine wirksame Menge einer Fluoridionenquelle umfasst.

## Revendications

1. Utilisation de polyéthylène glycol 600 (PEG 600) en association avec du propylène glycol en quantités respectives pour améliorer la stabilité rhéologique d'une composition de dentifrice, dans laquelle la composition comprend
un complexe de chlorure de zinc et de lysine, et
du PEG 600 en une quantité de 2 à 4 % en poids de la composition de dentifrice et
du propylène glycol en une quantité de 3 à 5 % en poids de la composition de dentifrice,
dans laquelle la composition comprend un acide organique ou inorganique pour réduire le pH, choisi parmi un ou plusieurs de l'acide phosphorique, de l'acide chlorhydrique, de l'acide sulfurique, de l'acide citrique, de l'acide tartrique, de l'acide malique, de l'acide maléique, de l'acide fumarique, de l'acide lactique, de l'acide gluconique et de l'acide benzoïque, en une quantité de 0,05 à 5 % en poids de la composition de dentifrice.

2. Utilisation selon la revendication 1, dans laquelle la composition comprend l'acide en une quantité de 0,1 à 2 % en poids de la composition de dentifrice.

3. Utilisation selon l'une quelconque des revendications précédentes, dans laquelle la lysine est libre ou sous forme de sel.

4. Utilisation selon l'une quelconque des revendications précédentes, dans laquelle le complexe de chlorure de zinc et de lysine de structure chimique [Zn(C₆H₁₄N₂O₂)₂Cl]⁺Cl⁻ (« 'ZLC ») est soit en solution du complexe cationique ([Zn(C₆H₁₄N₂O₂)₂Cl]⁺) et de l'anion chlorure, soit sous forme de sel solide, éventuellement sous forme monohydratée ou dihydratée.

5. Utilisation selon l'une quelconque des revendications précédentes, dans laquelle le complexe d'acide aminé et de zinc est présent en une quantité de 2 à 10 % en poids de la composition de dentifrice.

6. Utilisation selon l'une quelconque des revendications précédentes, dans laquelle le PEG 600 est présent en une quantité de 3 % en poids de la composition de dentifrice et le propylène glycol en une quantité de 4 % en poids de la composition de dentifrice.

7. Utilisation selon l'une quelconque des revendications précédentes, dans laquelle la composition comprend du tripolyphosphate de sodium.

8. Utilisation selon l'une quelconque des revendications précédentes, dans laquelle la composition comprend des ingrédients choisis parmi un ou plusieurs abrasifs, agents tampons, tensioactifs, épaississants, bandes ou fragments de gomme, rafraîchisseurs d'haleine, arômes, parfums, colorants, agents antibactériens, agents de blanchiment, agents qui interfèrent avec ou préviennent la fixation bactérienne, sources de calcium, sels de potassium oralement acceptables et polymères anioniques.

9. Utilisation selon l'une quelconque des revendications précédentes, dans laquelle la composition comprend une quantité efficace d'une source d'ions fluorure.
